# EUROPÄISCHE PATENTANMELDUNG

(11) **EP 1 420 248 A2**
(43) Veröffentlichungstag der Anmeldung: **19.05.2004**
(21) Anmeldenummer: 03024451.1
(22) Anmeldetag: 23.10.2003
(51) Int. Cl.: G01N 33/48, C12Q 1/68, B01L 3/00

(54) **Validiertes Design für Mikroarrays**

(30) Priorität: 18.11.2002 EP 02025820
(71) Anmelder: Schott Glas, 55122 Mainz (DE)
(72) Erfinder: van den Broek, Dirk, 55257 Budenheim (DE); Tudor, Jonathan, Worcestershire WR 135nd (GB); Schnabel, Roland, 65719 Hofheim (DE); Scholler, Patrick, 55122 Mainz (DE); Conzone, Samuel D., 55124 Mainz (DE)
(74) Vertreter: Schnabel, Jörg, Dr.

(57) **Zusammenfassung**

Die vorliegende Erfindung betrifft ein System zur Herstellung eines experimentell validierten Mikroarrays, insbesondere experimentell validierter DNA-Mikroarrays, ein entsprechendes Herstellungsverfahren. Das erfindungsgemäße System und das Verfahren zeichnen sich insbesondere durch eine Entkopplung von Bereitstellung des Layouts für den validierten Mikroarray und Herstellung des Endprodukts selbst aus.

## Beschreibung

Die vorliegende Erfindung betrifft ein System zur Herstellung eines experimentell validierten Mikroarrays, insbesondere experimentell validierter DNA-Mikroarrays, ein entsprechendes Herstellungsverfahren sowie den durch das erfindungsgemäße Verfahren erhältlichen Mikroarray. Das erfindungsgemäße System und das Verfahren zeichnen sich insbesondere durch eine Entkopplung von Bereitstellung des Layouts für den validierten Mikroarray und Herstellung des Endprodukts selbst aus.

Der Begriff "Mikroarray" bezeichnet in der Biotechnologie die geordnete Anordnung einer Vielzahl von Biomolekülproben auf möglichst geringem Raum. Mikroarrays dienen generell dazu, die Wechselwirkung von Probenmolekülen, deren Identität und/oder Menge unbekannt ist, mit einer möglichst großen Anzahl von potentiell mit dem zu untersuchenden Probenmolekül interagierenden bekannten Molekülen zu untersuchen. Im folgenden werden gemäß der Nomenklatur von Phimister (1999) Nature Genet. 21, Suppl.: 1-60, die zu untersuchenden Moleküle "Zielmoleküle" genannt, während die in der geordneten Anordnung vorliegenden bekannten Moleküle als "Sondenmoleküle" bezeichnet werden. Die Anordnung der Sondenmoleküle erfolgt im allgemeinen auf einem Glasträger, es werden jedoch auch Träger aus Kunststoff, Nylon oder Edelmetallen, wie Gold, verwendet.

Bei einem Mikroarray weisen die Spots mit den Sondenmolekülen typischerweise eine Größe von weniger als 200 µm im Durchmesser auf, und üblicherweise sind in derartigen Arrays Tausende von Spots mit Sondenmolekülen angeordnet.

Mikroarrays werden für eine Vielzahl von Sonden-/Zielmolekül-Paare und innerhalb der jeweiligen Sonden-/Zielmolekül-Kombinationen für unterschiedlichste Anwendungen verwendet. So gibt es bspw. Mikroarrays, bei denen verschiedene Peptide als Sondenmoleküle angeordnet sind, um die Wechselwirkung mit Zielproteinen zu untersuchen. Weiterhin können Proteinsonden immobilisiert sein, um die Bindung mit verschiedenen potentiellen Liganden zu untersuchen. Es ist jedoch auch möglich, umgekehrt eine Vielzahl von potentiellen organischchemischen niedermolekularen Liganden im Array anzuordnen und ein bestimmtes Zielprotein, bspw. ein zu inhibierendes Enzym, auf Bindung mit den potentiellen Liganden zu testen. Weitere Mikroarray-Systeme verwenden auch entsprechende Systeme mit Zuckermolekülen.

Das häufigste Array-System bezieht sich jedoch auf die Untersuchung von Wechselwirkungen zwischen Nukleinsäuren oder Analogen davon, z.B. Peptidnukleinsäuren. Bei diesem Typ sind im allgemeinen relativ kurze, bspw. < 100 Nukleotide umfassende, Oligonukleotide im Array angeordnet, während unbekannte oder in unbekannter Menge vorliegende Zielnukleinsäuren hinsichtlich einer Hybridisierung entsprechend den bekannten Basenpaarungsregeln getestet werden sollen.

Die Anwendungsmöglichkeiten, insbesondere der DNA-Mikroarray-Technologie, reichen von der Entdeckung von Genen, der Genotypisierung (z.B. Untersuchungen bezüglich Einzelnukleotid-Polymorphismen (SNP, engl. "Single Nucleotide Polymorphism")) über die Krankheitsdiagnose, die Arzneistoffentdeckung ("Pharmacogenomics") bis zur Forschung im toxikologischen Bereich ("Toxicogenomics") (vgl. auch die diesbezüglichen Ausführungen in WO 01/31333).

Die Erstellung eines für die jeweilige Fragestellung geeigneten Mikroarrays erfordert neben der prinzipiellen Ausgestaltung des Mikroarray-Systems, umfassend die Auswahl der Sonden, die Herstellung des anfänglichen Arrays auf einem Träger (Substrat oder umgangssprachlich "Chip"), die Bereitstellung der Zielmoleküle, die Durchführung des Tests, die Datenaufnahme und die Verarbeitung der Daten, nach dem Durchlaufen all dieser Schritte vor allem die Validierung des Mikroarray-Systems, d.h. die nachfolgende Überprüfung, ob die zunächst hergestellte Anordnung der Sondenmoleküle die Zielmoleküle im Probenmaterial entsprechend spezifisch bindet, um bspw. die Funktionsweise potenzieller Wirkstoffe tatsächlich identifizieren zu können.

Diese Validierung ist bei jedem bereitzustellenden Mikroarray-System erforderlich, da bei der Herstellung jedes gegebenen Mikroarrays zum einen oft Fehler bei der Synthese und/oder beim Aufbringen der Sondenmoleküle auftreten, zum anderen insbesondere bei DNA-Mikroarrays durch Computer gesteuerte *in silico* Verfahren nicht die optimalen Oligonukleotide ausgewählt werden können und insbesondere auch die Position im Gen, die Länge und Menge der in jedem Spot befindlichen Sondenmoleküle für die gegebene Anwendung angepaßt werden muß, um ein möglichst optimales Signal zu ergeben.

Im Stand der Technik sind einige Systeme und Verfahren zur Herstellung von Mikroarrays bekannt. Dabei wird im allgemeinen nach zwei verschiedenen Vorgehensweisen verfahren: Bei einem Verfahren werden die Sondenmoleküle *in situ* (z.B. mit Hilfe entsprechender photolithographischer Maskenbelichtungsverfahren) auf dem jeweiligen Substrat synthetisiert. Bei einer anderen Vorgehensweise werden die Sondenmoleküle zunächst *ex situ* durch ein geeignetes Syntheseverfahren (z.B. übliche Festphasensynthesen) hergestellt und danach auf das jeweilige Substrat aufgebracht (bspw. durch sog. "Spotting" bzw "Printing").

Die Herstellung eines validierten, d.h. möglichst fehlerfreien, Mikroarrays, mit Hilfe der Synthese *ex situ* und nachfolgendem Spotting dauert sehr lange, in der Regel mehrere, meist in der Größenordnung von weit mehr als 5 Wochen, bspw. bis zu 5 Monaten, da das anfängliche Design des Mikroarrays bei der Validierung in mehreren Bewertungsrunden an die gegebene Fragestellung angepaßt werden muß. Die Herstellung vieler verschiedener Sondenmolekülspezies (sog. "Features") und deren Spotting für die jeweils angepasste Ausgestaltung des Arrays in jeder Bewertungsrunde ist somit hinsichtlich der Dauer vom anfänglichen bis zum validierten Array nachteilig. In Anbetracht der Tatsache, dass sich derzeit ca. 600 Genome in der finalen Sequenzierungsphase befinden und die Masse der Geninformation weiterhin drastisch zunehmen wird, wird ein wesentlich schnelleres Verfahren zur Genfunktionsuntersuchung mit Hilfe von Mikroarrays zunehmend unverzichtbar. Außerdem werden die Anwendungen mit zunehmendem Kenntnisgewinn spezifischer und der Zeitdruck für die Mikroarray-Entwicklung größer. Ein zu langer Zeitraum ist insbesondere bei Wirkstoffuntersuchungen, bei denen eine schnellere Anpassung wünschenswert ist, besonders nachteilig. Insbesondere in der Diagnostik wird häufig eine schnelle patientenspezifische Bewertung möglicher Wirkstoffe oder Wirkstoffkombinationen erwartet. Demgegenüber sind *in situ* Verfahren und entsprechende Systeme mit geeigneten Vorrichtungen zur Mikroarray-Herstellung, wenn viele verschiedene Features aufgebracht werden sollen, grundsätzlich überlegen. Die *in situ* Verfahren sind jedoch dahingegehend nachteilig, dass sie insbesondere bei der Massenproduktion eines validierten Mikroarrays gegenüber der vorstehend genannten Vorgehensweise *(ex situ* Synthese/Spotting) apparativ erheblich aufwendiger und teurer sind.

Der vorliegenden Erfindung liegt daher die Aufgabe zugrunde, eine möglichst schnelle und kostengünstige Herstellung von validierten Mikroarrays im industriellen Produktionsmaßstab zu ermöglichen.

Diese Aufgabe wird durch die in den Ansprüchen gekennzeichneten Ausführungsformen der vorliegenden Erfindung gelöst.

Insbesondere wird erfindungsgemäß ein System zur entkoppelten Herstellung validierter Mikroarrays mit mindestens
- einer oder mehreren Einrichtung(en), die zur Eingabe von Informationen über durch einen Mikroarray zu analysierende Zielmoleküle ausgelegt ist/sind,
- einer Einrichtung, die zum Ermitteln von potentiell mit den Zielmolekülen wechselwirkenden Sondenmolekülen (Testsondenmoleküle) anhand von Informationen über zu analysierende Zielmoleküle ausgelegt ist,
- einer Layout-Bereitstellungseinrichtung, umfassend
   - eine Vorrichtung zur *in situ* Synthese von Testsondenmolekülen in hoher Dichte,
   - eine Mikroarray-Testvorrichtung, die zum Inkontaktbringen von Zielmolekülen mit Testsondenmolekülen ausgelegt ist, und
   - eine Mikroarray-Auslesevorrichtung, die zur Aufnahme von beim Inkontaktbringen von Zielmolekülen und Testsondenmolekülen veränderlichen Signalen ausgelegt ist,
- einer Sondenmolekülsynthese-Einrichtung, die zur *ex situ* Synthese von Sondenmolekülen ausgelegt ist, und
- einer Mikroarray-Bereitstellungseinrichtung, die zum Aufbringen von Sondenmolekülen auf Substrate ausgelegt ist,
bereitgestellt.

Das erfindungsgemäße System ist vorzugsweise durch Rechner-gestützte Technologien ausgestaltet. Gemäß dieser bevorzugten Ausführungsform umfasst bspw. jede Einrichtung zur Eingabe von Informationen über von durch einen Mikroarray zu analysierende Zielmoleküle einen (oder mehrere) Rechner, der (die) die Informationen in einem oder mehreren Daten-File(s) ablegt (ablegen). Desgleichen umfasst vorzugsweise die Einrichtung zur Ermittlung von Testsondenmolekülen einen Rechner, der die Testsondenmoleküle programmgesteuert anhand von Informationen über Zielmoleküle auswählt. Darüber hinaus umfasst vorzugsweise auch die vorstehende Layout-Bereitstellungseinrichtung einen Rechner (gegebenenfalls auch mehrere), der (die) das Layout in Form eines oder mehrerer Daten-File(s) ablegt (ablegen). Bspw. kann die Layout-Bereitstellungseinrichtung einen (oder wiederum mehrere) Daten-File(s) (im geeigneten Format) erzeugen, der (die) Informationen für die von der obigen Sondenmolekülsynthese-Einrichtung herzustellenden Sondenmoleküle enthält (enthalten), während ein anderer (oder mehrere andere) Daten-File(s) erzeugt wird (werden), der (die) Informationen über die Art und Weise der Aufbringung, die Dichte, Anzahl, Orientierung, Anordnung usw. auf dem Mikroarray (für die Mikroarray-Bereitstellungseinrichtung) enthält (enthalten). Erfindungsgemäß verwendbare Rechner und entsprechende Steuerprogramme sind einem Fachmann bekannt.

Die Layout-Bereitstellungseinrichtung der vorliegenden Erfindung dient der Ermittlung der Ausgestaltung des herzustellenden validierten Mikroarrays. Der Begriff "Layout" des Mikroarrays betrifft erfindungsgemäß das Design, d.h. die einen Mikroarray kennzeichnenden Parameter, wie Art, Beschaffenheit (insbesondere Sequenz von Oligonukleotiden, Peptiden usw.), Menge, Dichte, Bindung (bspw. welche(r) Linker zur Bindung der Sondenmoleküle auf dem für den Mikroarray verwendeten Träger eingesetzt wird bzw. werden), Orientierung (bspw. 5' nach 3' bzw. umgekehrt bei Nukleinsäuren, N-terminal nach C-terminal bzw. umgekehrt im Fall von Peptiden, Polypeptiden bzw. Proteinen usw.) und Anordnung von Sondenmolekülen auf dem Mikroarray.

Die Layout-Bereitstellungseinrichtung umfasst eine Vorrichtung zur *in situ* Synthese von Testsondenmolekülen in hoher Dichte. Derartige Vorrichtungen zeichnen sich dadurch aus, dass eine hohe Anzahl, bspw. mehr als 1000, vorzugsweise mindestens 4000, verschiedene (Test-)Sondenmolekülspezies, direkt auf dem für den Mikroarray gewählten Substrat (also *in situ)* auf einer für Mikroarrays typischen Fläche, von bspw. etwa 1200 mm² bis etwa 3000 mm², synthetisiert werden, wobei bevorzugte Mikroarray-Ausgestaltungen bspw. Maße von etwa 20 x 60 mm bis etwa 38 x 75 mm, vorzugsweise etwa 25 x 75 mm, aufweisen.

Eine "(Test-)Sondenmolekülspezies" bezeichnet dabei jeweils ein Sondenmolekül spezifischer Struktur, bspw. eine Nukleinsäure, insbesondere DNA, bestimmter Nukleotidsequenz oder ein (Poly- oder Oligo-)Peptid mit einer bestimmten Aminosäuresequenz. Eine (Test-)Sondenmolekülspezies wird im allgemeinen auch als "Feature" bezeichnet.

Entsprechende *in situ* Synthese-Vorrichtungen sind einem Fachmann bekannt, und sind üblicherweise photolithographisch hergestellte Belichtungsvorrichtungen oder Vorrichtungen, die nasschemisch im Druckverfahren bzw. elektrochemisch arbeiten.

Insbesondere zur Herstellung validierter DNA-Mikroarrays sind erfindungsgemäß verwendbare Vorrichtungen zur nasschemischen *in situ* Synthese im Druckverfahren z.B. bei Clondiag Chip Technologies GmbH (Jena, Deutschland) erhältlich. Photolithographie-Vorrichtungen, bspw. zur DNA-Synthese *in situ,* sind erfindungsgemäß bevorzugt und werden z.B. von NimbleGen Systems, Inc. (Madison, WI, USA), Affymetrix, Inc. (Santa Clara, CA, USA), Xeotron Corp. (Houston, TX, USA), Combinature Biopharm AG (Berlin, Deutschland) und Febit AG (Mannheim, Deutschland) angeboten. Besonders bevorzugt ist aufgrund der Möglichkeit, eine besonders hohe Anzahl bzw. Dichte an (Test-)Sondenmolekülen bei geringen Kosten und in kurzer Zeit *in situ* zu synthetisieren, eine Vorrichtung zur MAS (engl. "Maskless Array System") Synthese der (Test-)Sondenmoleküle. Hiermit können routinemäßig über 400.000 und in speziellen Anordnungen z.B. 750.000 (DNA)-Features in einem Array synthetisiert werden. Die MAS-Technik ist z.B. in WO 99/42813 beschrieben, deren Offenbarungsgehalt hiermit vollständig in die vorliegende Erfindung eingeschlossen ist. Weitere geeignete Vorrichtungen zur MAS-Synthese und besondere Ausgestaltungen dieser Technik sind in WO 01/34847 und WO 02/04597 dargelegt, deren diesbezügliche Offenbarungsgehalte ebenfalls durch Bezugnahme in die vorliegende Erfindung aufgenommen sind. Weitere bevorzugte *in situ* Synthese-Vorrichtungen sind elektroschemisch und mit *in situ* Spotting arbeitende Vorrichtungen.

Die weiteren in der Layout-Bereitstellungseinrichtung enthaltene Vorrichtungen, wie Mikroarray-Test- und -Auslesevorrichtung, sind ebenfalls Stand der Technik und im Handel erhältlich. Entsprechende Hersteller, die auch komplett integrierte Vorrichtungen anbieten, sind z.B. Agilent (USA), Genomic Solutions (USA), Affymetrix (USA), Axon Instruments, Inc. (USA) und Packard Bioscience (USA).

Die ex situ Sondenmolekülsynthese-Einrichtung des erfindungsgemäßen Systems dient der Synthese der Sondenmoleküle für den als Endprodukt bereitzustellenden Mikroarray und ist vorzugsweise eine im Stand der Technik bekannte Festphasensynthese-Vorrichtung. Vorrichtungen zur *ex situ* Festphasensynthese von Sondenmolekülen, z.B. Oligonukleotiden, insbesondere zur Festphasensynthese nach Art der Merrifield-Synthese (H.G. Gassen et al. (1982) Chemical and Enzymatic Synthesis of Genefragments, Verlag Chemie, Weinheim; Gait (1987) Oligonucleotide synthesis: a practical approach, IRL Press, Oxford) oder auf andere Art (Beyer und Walter (1984) Lehrbuch der organischen Chemie, Seite 816 ff., 20. Auflage, S. Hirzel Verlag, Stuttgart) sind allgemein bekannt und werden bspw. von Dharmacon Recearch, Inc. (USA), und Synthegen (USA) angeboten. Üblicherweise werden Sondenmolekül-Festphasensynthesen, insbesondere im Fall von Oligonukleotiden, als Service, z.B. von Proligo (USA), Sigma (Deutschland), MWG Biotech AG (Deutschland) u.a., angeboten. Derartige Festphasensynthese-Vorrichtungen weisen den Vorteil auf, dass ein gegebenes Sondenmolekül bzw. eine gegebene Sondenmolekülspezies (Feature), z.B. ein Oligonukleotid gegebener Sequenz etc., in großer Menge bei niedrigen Kosten in relativ kurzer Zeit und guter Ausbeute bereitgestellt werden kann.

Mit der Mikroarray-Bereitstellungseinrichtung des erfindungsgemäßen Systems werden *ex situ* synthetisierte Sondenmoleküle auf ein entsprechendes Substrat aufgebracht, um derart einen Mikroarray fertig zu stellen. Erfindungsgemäß verwendbare Vorrichtungen zum Aufbringen von *ex situ* synthetisierten Sondenmolekülen sind ebenfalls Stand der Technik und im Handel, bspw. bei Biorobotics (USA), Genemachines (USA), Perkin Eimer Life Sciences (USA), MWG Biotech AG (Deutschland) oder GeneScan Europe AG (Deutschland) erhältlich. Erfindungsgemäß bevorzugte Vorrichtungen zum Aufbringen von *ex situ* synthetisierten Sondenmolekülen sind entsprechende Spotting- bzw. Printing-Vorrichtungen.

Die vorstehend angegebenen Komponenten des Systems der vorliegenden Erfindung sind vorzugsweise Rechner-gesteuert und werden somit halb- oder vollautomatisch betrieben. Die Komponenten, insbesondere die entsprechenden Layout-Bereitstellungseinrichtung, die Sondenmolekülsynthese-Einrichtung und die Mikroarray-Bereitstellungseinrichtung sind typischerweise mit im Stand der Technik bekannten und allgemein verfügbaren Mikrofluid- und mikromechanischen Komponenten ausgerüstet.

Daher sind erfindungsgemäß bevorzugte Ausgestaltungen der im System der vorliegenden Erfindung eingesetzten Einrichtungen des Weiteren mit Computern ausgestattet, welche die Daten-Eingabe und -Verarbeitung wesentlich vereinfachen. Im System sind die Komponenten für den erforderliche Datenaustausch (z.B. zwischen Eingabe-Einrichtung und Layout-Bereitstellungseinrichtung, zwischen Layout-Bereitstellungseinrichtung und Sondenmolekülsynthese-Einrichtung sowie Mikroarray-Bereitstellungseinrichtung) vorzugsweise über Datenübertragungsvorrichtungen in einem Netzwerk verbunden. Das Netzwerk kann z.B. ein lokales Netzwerk sein, in dem bspw. nur wenige Rechner miteinander kommunizieren. Vorteilhafterweise handelt es sich bei dem Netzwerk um das Internet. Hinsichtlich der grundsätzlichen Ausgestaltung des "World Wide Web" und der Kommunkationsalgorithmen wird bspw. auf die diesbezüglichen Ausführungen in WO 01/31333 verwiesen. Es kann sich bei dem Netzwerk auch um irgendeinen anderen regionalen, nationalen oder internationalen Zusammenschluß von über Datenübertragungseinrichtungen kommunizierenden Computern handeln.

Unter dem Gesichtspunkt des Verbundes der erfindungsgemäßen Einrichtungen in einem Netzwerk, z.B. über das Internet, bildet eine weitere bevorzugte Ausgestaltung der vorliegenden Erfindung ein System, in welchem die Einrichtungen eine Plattform für die Interaktion (Kommunikation, gegenseitiger Datenaustausch usw.) in einem Forschungsverbund oder -konsortium bereitstellen. In derartigen Forschungskonsortien, die insbesondere auch delokalisiert an gemeinsamen Projekten arbeiten, können nach der Nutzung der experimentell validierten Mikroarrays aufgrund des Wissenszuwachses neue Anforderungen an die Zusammenstellung (d.h. das Layout) des Mikroarrays dazu führen, dass Re-Designs erforderlich sind. Mit Hilfe des delokalisierten Designs (Ermittlung des Layouts) und einer Produktion des validierten Mikroarrays vor Ort kann so die Effizienz der Wissenssteigerung drastisch erhöht werden. Eine Ausführungsform zur effizienten Nutzung eines derartigen Plattform-Systems bildet z.B. ein Web-basisertes (insbesondere Internet-basiertes) Laborinformationsmanagementsystem (LIMS), an dem alle Beteiligten (z.B. die Mitglieder eines Forschungsverbundes oder - konsortiums) gemeinsam Mikroarray-Layouts, re-designte Layouts und Mikroarrays verwalten. Ein Beispiel für ein derartiges LIMS ist "Partisan" von Clondiag.

Ein weiterer Gegenstand der vorliegenden Erfindung betrifft ein Verfahren zur entkoppelten Herstellung eines validierten Mikroarray-Layouts, insbesondere mit dem vorstehend definierten System, das die folgenden Schritte umfaßt:
(a) Bereitstellen von Informationen über durch den Mikroarray zu analysierende Zielmoleküle, vorzugsweise durch die obige Eingabe-Einrichtung,
(b) Ermitteln von Testsondenmolekülen (d.h. von potentiell mit den Zielmolekülen wechselwirkenden Sondenmolekülen) anhand der Informationen, bspw. durch die erfindungsgemäße Einrichtung zur Ermittlung von Testsondenmolekülen,
(c) Bereitstellen eines validierten Layouts für den Mikroarray, insbesondere durch die vorstehend definierte Layout-Einrichtung, umfassend das
   - Herstellen mindestens eines Testmikroarrays durch *in situ* Synthese der ermittelten Testsondenmolekülen in hoher Dichte, vorzugsweise durch die obige *in situ* Synthese-Vorrichtung,
   - Testen des Testmikroarrays mit Zielmolekülen, vorzugsweise durch die obige Mikroarray-Testvorrichtung, und
   - Aufnahme mindestens eines beim Testen des Testmikroarrays mit den Zielmolekülen veränderlichen Signals, insbesondere durch die obige Mikroarray-Auslesevorrichtung,
(d) Synthetisieren der dem validierten Layout entsprechenden Sondenmoleküle (validierte Sondenmoleküle) *ex situ,* insbesondere durch die vorstehende Sondenmolekülsynthese-Einrichtung, und
(e) Aufbringen der validierten Sondenmoleküle gemäß dem validierten Layout auf ein Substrat, vorzugsweise durch die erfindungsgemäße Mikroarray-Bereitstellungseinrichtung.

Das erfindungsgemäße System und das Verfahren beruhen auf der Erkenntnis, dass der für die Bereitstellung eines validierten Mikroarray-Layouts erforderliche Prozeß der Herstellung von einem oder mehreren Testmikroarrays durch ein *in situ* Verfahren mittels einer entsprechenden Vorrichtung durchgeführt wird, während die Herstellung des eigentlichen (validierten) Mikroarrays durch *ex situ,* vorzugsweise Festphasensynthese der (dem validierten Layout entsprechenden) Sondenmoleküle und nachfolgend deren Aufbringung ("Spotting"), wiederum mit Hilfe der geeigneten Systemkomponenten, auf das geeignete Substrat erfolgt. Daher ist beim erfindungsgemäßen System bzw. beim Verfahren der Validierungsprozeß von der Herstellung des Endprodukts, des validierten Mikroarrays, entkoppelt. Durch die erfindungsgemäße Kombination werden die zwei genannten Verfahrensprinzipien optimal miteinander derart verbunden, dass jedes Verfahrensprinzip dort eingesetzt wird, wo dessen jeweiligen spezifischen Vorteile zur Geltung kommen: Die *in situ* Synthese in hoher Dichte ist besonders flexibel und eignet sich daher zur Herstellung von Testmikroarrays, die von einem möglichst großen Pool von potentiell mit den Zielmolekülen wechselwirkenden (Test-)-Sondenmolekülen ausgehen. Steht das validierte Layout des Mikroarrays bereit, so erfolgt dessen Herstellung durch die schnelle und kostengünstige *ex situ* Synthese der Sondenmoleküle und das nachfolgende Aufbringen auf das Substrat, jeweils gemäß dem validierten Layout. Da die Sondenmoleküle durch den Validierungsprozess bereits auf eine relativ geringe Anzahl begrenzt sind, müssen bei der *ex situ* Synthese für das Endprodukt nur vergleichsweise wenige verschiedene Sondenmolekülspezies hergestellt werden, weshalb sich mit Hilfe des erfindungsgemäßen Verfahrens im Vergleich zu bisher üblichen Vorgehensweisen die Herstellungskosten für einen validierten Mikroarray deutlich senken lassen. Die ex situ Synthese der Sondenmoleküle für das Endprodukt eignet sich insbesondere für die Mikroarray-Produktion im industriellen Maßstab, weshalb sich die Vorteile des erfindungsgemäßen Verfahrens ganz besonders dort zeigen, wo eine große Anzahl eines validierten Mikroarrays benötigt wird. Im erfindungsgemäßen Herstellungsverfahren wirken daher die genannten Vorgehensweisen synergistisch zusammen, wodurch es ermöglicht wird, einen validierten Mikroarray schnell, kostengünstig und mit geringer Fehlerquote bereitzustellen.

Erfindungsgemäß wird das Verfahren bevorzugt derart durchgeführt, dass der Schritt (a) z.B. bei einem oder mehreren Mikroarray-Anwender(n) durchgeführt wird, die Schritte (b) und (c) räumlich getrennt davon z.B. bei einem Layout-Dienstleister) durchgeführt werden, der Schritt (d) z.B. bei einem Sondenmolekül-Hersteller und der Schritt (e) z.B. bei einem Mikroarray-Hersteller durchgeführt wird. Selbstverständlich ist es auch möglich, die Schritte (b) und (c) jeweils räumlich getrennt voneinander durchzuführen. So kann bspw. die Ermittlung der Testsondenmoleküle auch vom Mikroarray-Anwender durchgeführt werden und dieser so die Testsondenmoleküle dem Layout-Dienstleister vorgibt.

Insbesondere bei der vorstehend dargelegten räumlichen Trennung der Verfahrensschritte, ist es erfindungsgemäß bevorzugt, den erforderlichen Informationsfluß durch Übertragung elektronischer Daten zu verwirklichen. Daher erfolgt die Bereitstellung der Informationen im Schritt (a) bevorzugt in Form eines Daten-Files mit Hilfe einer elektronischen Datenübertragungseinrichtung. Ebenso erfolgt die Bereitstellung des validierten Layouts im Schritt (c) bevorzugt in Form eines Daten-Files mit Hilfe einer elektronischen Datenübertragungseinrichtung. So werden bspw. vom Anwender die Informationen über die Zielmoleküle in elektronischer Form in die erfindungsgemäße Eingabe-Einrichtung eingegeben und zur Einrichtung zur Ermittlung der Testsondenmolekülke, die bspw. bei einem Layout-Dienstleister angeordnet ist, übertragen. Dieser wiederum überträgt nach Erstellung des validierten Layouts des Mikroarrays die Informationen über die (validierten) Sondenmoleküle, gegebenenfalls das vollständige Layout, umfassend die vorstehend angegebenen weiteren, den validierten Mikroarray kennzeichnenden Daten, als Daten-File(s) zur Sondenmolekülsynthese-Einrichtung (vorzugsweise bei einem Sondenmolekül-Hersteller angeordnet). Des Weiteren werden die validierten Layout-Daten der Mikroarray-Bereitstellungseinrichtung (die bspw. bei einem entsprechenden Mikroarray-Hersteller angeordnet ist) bevorzugt in Form eines bzw. mehrerer Daten-Files bereitgestellt.

Hierzu sind die im erfindungsgemäßen System befindlichen Komponenten, wie bereits vorstehend dargelegt, vorteilhaftererweise durch ein Computer-Netzwerk, inbesondere per Internet, miteinander verbunden. Möglichkeiten der Kommunikation zwischen Bereitstellungs-Einrichtung (Schritt (a)), Ermittlung-Einrichtung (Schritt (b), Layout-Bereitstellungseinrichtung (Schritt (c)), Sondenmolekülsynthese-Einrichtung (Schritt (d)) und Mikroarray-Bereitstellungseinrichtung (Schritt (e)), insbesondere über das Internet, vorzugsweise unter Verwendung einer entsprechenden System Software sind Stand der Technik und z.B. in WO 01/31333 dargelegt, deren diesbezüglicher Offenbarungsgehalt vollumfänglich in die vorliegende Erfindung eingeschlossen ist.

Im Schritt (a) des erfindungsgemäßen Verfahrens werden zunächst dem System Informationen über durch den Mikroarray, dessen Layout bereitgestellt werden soll, zu analysierende Zielmoleküle, d.h. deren kennzeichnende Eigenschaften, bereitgestellt. Diese Informationen beinhalten bspw. die Art und Beschaffenheit der Zielmoleküle, z.B. ob es sich bei den zu analysierenden Zielmolekülen um Nukleinsäuren, Peptide (darunter Oligo-, Polypeptide sowie auch Proteine) Peptidnukleinsäuren, niedermolekulare organische Substanzen, Saccharide (z.B. Oligo- und Polysaccharide) usw. (wobei auch Gemische der genannten Molekülspezies vorliegen können) handelt. Des Weiteren umfassen diese Informationen Angaben über die Oberflächenstruktur, das Volumen (was bspw. auch Angaben über sterische Verhältnisse einschließt) usw. der Zielmoleküle und/oder ggf. des für den Mikroarray zu verwendenden Träger- bzw. Substratmaterials, über das Haftoder Bindungsverhalten der Zielmoleküle, einschließlich über potentiell unspezifische Wechselwirkungen gegenüber möglicherweise einzusetzenden Sondenmolekülen und/oder einem geplanten Trägermaterial, wobei diese Bindungsparameter insbesondere Angaben über die Kinetik des Bindungsverhaltens, wie Bindungskonstanten, kooperative Parameter usw., umfassen und auch ggf. vorhandene Strukturflexibilitäten berücksichtigen. Weitere Angaben beziehen sich auf die bei einem mit dem Mikroarray durchzuführenden Experiment vorhandenen Reaktionsbedingungen, wie Temperatur, Pufferbedingungen (pH, Salzgehalt, Detergentien, Hilfsstoffe usw.). Aus diesen Informationen ergeben sich anhand der dadurch zu Verfügung stehenden Problemstellung die auf dem Mikroarray aufzutragenden Sondenmoleküle, welche potentiell mit den Zielmolekülen wechselwirken. Diese zunächst ermittelten Sondenmoleküle müssen jedoch durch Anpassen der Mikroarray-Ausgestaltung in einem Validierungsprozeß hinsichtlich ihrer tatsächlichen Eignung für den herzustellenden Mikroarray überprüft werden. Daher sind diese zunächst ermittelten Sondenmoleküle erfindungsgemäß als "Testsondenmoleküle" gekennzeichnet.

Wie bereits vorstehend ausgeführt, werden die Informationen über die Zielmoleküle, insbesondere das dem bereitzustellenden Layout zugrunde liegende Analyseproblem, und/oder das validierte Layout in Form von Daten-Files mit Hilfe einer elektronischen Datenübertragungseinrichtung bereitgestellt. Eine derartige Datenübertragungseinrichtung besteht bspw. aus zwei z.B. miteinander per E-Mail kommunizierenden Computern. Dabei wird typischerweise der Anwender von einem dezentral angeordneten Rechner aus vorzugsweise per elektronischer Datenübertragung, bspw. per E-Mail, die benötigten Informationen hinsichtlich der Zielmoleküle einem Zentralrechner übertragen. Die Informationen können selbstverständlich auch auf allen möglichen anderen Datenträgern, bspw. Disketten, CD-ROM usw. zur Verfügung gestellt werden. Die jeweiligen Datensätze können dann bspw. mit Datensätzen bereits erstellter Mikroarrays bzw. Mikroarray-Layouts verglichen werden (z.B. aus zuvor durchgeführten Untersuchungen, die als "Trainingssätze" zur weiteren Optimierung eingesetzt werden können), um dadurch ein "voroptimiertes" Mikroarray-Layout zu ermitteln. Zur Optimierung werden die Datensätze zunächst vorzugsweise in ein einheitliches Format überführt und dann über entsprechend aus diesen formatierten Daten erstellten Matrizen und/oder mit Hilfe von neuronalen Netzen mit bereits vorhandenen Datensätzen verglichen und ggf. entsprechend angepasst.

Das erfindungsgemäße Verfahren ist hinsichtlich der Art der Test- oder validierten Sondenmoleküle bzw. der Zielmoleküle nicht eingeschränkt. Somit kann es sich bei diesen bspw. um Nukleinsäuren, Peptide (darunter Oligo-, Polypeptide sowie auch Proteine, insbesondere Antikörper oder Fragmente davon), Peptidnukleinsäuren, niedermolekulare organische Substanzen, Saccharide (z.B. Oligo- und Polysaccharide) usw. handeln, wobei auch Gemische der genannten Molekülspezies vorliegen können.

Das erfindungsgemäße Herstellungsverfahren eignet sich ganz besonders zur Validierung von Mikroarrays, die der Untersuchung von Nukleinsäuren als Zielmolekülen dienen. Daher wird im folgenden das erfindungsgemäße Verfahren beispielhaft anhand dieser Variante beschrieben. Es ist allerdings für einen Fachmann klar, dass das dem erfindungsgemäßen Verfahren zurunde liegende Gesamtkonzept auch auf alle möglichen anderen Mikroarray-Problemstellungen, insbesondere auf andere, strukturell unterschiedliche Sonden- und Zielmoleküle, zwanglos übertragbar ist.

Im Falle eines DNA-Mikroarrays handelt es sich somit bei den im Schritt (a) bereitzustellenden Informationen über die Zielmoleküle typischerweise um die dem Mikroarray-Test zugrunde liegende genomische Problemstellung. Dies umfaßt beispielsweise die Information darüber, aus welchem Organismus die zu analysierenden Zielmoleküle, hier also DNA, stammen. Es kann sich bei den Informationen über die zu analysierenden DNA-Zielmoleküle jedoch auch um weitergehende Angaben handeln, welche die zu analysierende Population von DNA-Molekülen genauer kennzeichnen. Insbesondere wird es sich dabei typischerweise um die Sequenz der Ziel-Nukleinsäuren handeln. Diese wird vorzugsweise in einem Daten-File bereitgestellt, wobei geeignete Formate einem Fachmann bekannt sind. Ein weithin bekanntes Standard-Datenformat für Sequenzen ist z.B. das FASTA-Format. Des Weiteren kann es sich bei den Informationen auch um Angaben über einen spezifischen Zelltyp, z.B. ob es sich um eine Krebszelle allgemein oder eine irgendwie sonst krankhafte Zelle handelt oder beispielsweise ob eine bestimmte Zellinie zu untersuchen ist usw. Weitere Angaben beziehen sich häufig auf dem durchzuführenden Mikroarray-Test vorausgehende Einwirkungen auf den Organismus, beispielsweise bestimmte Zellen, Organe, Organteile usw., Ereignisse oder Behandlungen, die bspw. im Aussetzen des Organismus oder eines Teils davon gegenüber bestimmten Substanzen, bspw. Arzneistoffen, toxikologisch relevanten Stoffen usw., bestehen.

Nachdem die Informationen über die zugrunde liegende Problemstellung, d.h. die Informationen über die zu analysierenden Zielmoleküle, bspw. einer Zentralstelle, wie bei einem Layout-Dienstleister angeordneten zentralen Rechner, vorzugsweise mittels einer Datenübertragungseinrichtung bereitgestellt wurden, werden im Schritt (b) des erfindungsgemäßen Verfahrens die potentiell mit den Zielmolekülen wechselwirkenden Testsondenmoleküle ermittelt. Dies erfolgt gemäß einer bevorzugten Ausführungsform mittels einem Fachmann geläufiger Computerprogramme, bspw. Arraydesigner 2 (Primer Biosoft International) u.v.a. Im bevorzugten Beispiel des erfindungsgemäßen Herstellungsverfahrens werden daher die anhand der (genomischen) Problemstellung im Schritt (b) ermittelten Sequenzen der als Testsondenmoleküle zu verwendenden Nukleinsäuren, insbesondere Oligonukleotide, die z.B. eine Länge von etwa 4 bis etwa 80, vorzugsweise etwa 8 bis etwa 80 Nukleotiden aufweisen, bereitgestellt.

Gemäß einer besonders bevorzugten Ausführungsform repräsentieren die bspw. mit Hilfe spezieller Computersoftware ermittelten Testsondenmoleküle im Schritt (b) eine Gesamtpopulation von Molekülen, die mit den Zielmolekülen, welche mit Hilfe des Mikroarrays, dessen Layout bereitzustellen ist, untersucht werden sollen, potentiell wechselwirken. Bei Oligonukleotiden als Sondenmolekülen in der *in situ* Synthese, insbesondere mit der MAS-Technologie, kann bspw. diese Gesamtpopulation das Gesamtgenom eines Organismus darstellen. Beispiele von Organismen sind z.B. Menschen, Tiere, wie Maus, Ratte, Ziege, Schwein, Rind, Meerschweinchen, *Danio rerio* und *C*. *elegans,* Pflanzen, wie Nutzpflanzen, Ara*bidopsis* etc., Protozoen, Bakterien, wie *E*. *coli,* Pilze, z.B. Hefen, wie *S*. *cerevisae,* schließen erfindungsgemäß aber auch Viren, Viroide usw. ein. Die Sondenmoleküle, insbesondere Oligonukleotide, können jedoch auch Teile einer größeren Gesamtpopulation repräsentieren. Im Fall von Oligonukleotiden kommen hier bspw. als (Teil-)Gesamtpopulationen das genetische Material eines oder mehrerer Chromosomen von Organismen, bspw. den vorstehend genannten, in Frage.

Weitere (Teil-)Gesamtpopulationen können einzelne cDNA-Bibliotheken sein. Des Weiteren können die Testsondenmoleküle Splice-Varianten (z.B. alle Splice-Varianten eines Primärtranskripts oder von Primärtranskripten einer bestimmten Gen-Gruppe oder -Untergruppe), Promotorregionen, SNPs (z.B. alle (bisher) bekannten SNPs eines bestimmten Gens oder einer Gen-Gruppe oder - Untergruppe) Mutationen (wie bei SNPs), hochrepetitive DNA-Bereiche usw. repräsentieren.

Vorzugsweise umfasst die Bereitstellung des validierten Layouts erfindungsgemäß die folgenden Unterschritte:
(1) Festlegen von mindestens einem bei einer Wechselwirkung der Zielmoleküle mit Sondenmolekülen von einem Mikroarray zu erwartenden Signal (Erwartungssignal),
(2) Herstellen eines Testmikroarrays durch *in situ* Synthese der ermittelten Testsondenmoleküle in hoher Dichte, vorzugsweise durch die vorstehend definierte *in situ* Synthese-Vorrichtung,
(3) Testen des Testmikroarrays mit Zielmolekülen, insbesondere durch die erfindungsgemäße Mikroarray-Testvorrichtung, wobei mindestens ein Testsignal aufgenommen wird, vorzugsweise unter Verwendung der vorstehend definierten Mikroarray-Auslesevorrichtung,
(4) Vergleichen des mindestens einen Testsignals mit dem mindestens einen Erwartungssignal,
(5) Anpassen des Testmikroarrays durch Wiederholung der Schritte (2) bis (4) bis das Testsignal im wesentlichen dem Erwartungssignal entspricht, wobei im Schritt (2) die Sondenmoleküle, deren Menge, Bindung, Anzahl, Dichte, Orientierung und/oder Anordnung auf dem Mikroarray verändert werden bzw. wird, und
(6) Bereitstellen des Mikroarray-Layouts, bei dem das mindestens eine Testsignal im wesentlichen dem mindestens einen Erwartungssignal entspricht.

Im Unterschritt (1) der bevorzugten Ausführungsform des erfindungsgemäßen Verfahrens werden ein oder mehrere Erwartungssignale festgelegt, das oder die bei einer Wechselwirkung der Zielmoleküle mit Sondenmolekülen von einem Mikroarray zu erwarten wäre(n). Derartige Signale können alle experimentell messbaren chemischen oder physikalischen Parameter sein, die mit Hilfe geeigneter Mikroarray-Auslesevorrichtungen (d.h. Messvorrichtungen) bei einem Test, bei dem Zielmoleküle mit Sondenmolekülen (unter Verwendung einer vorstehend definierten Mikroarray-Testvorrichtung) in Kontakt gebracht werden, aus einem entsprechenden Mikroarray ausgelesen werden können. Vorzugsweise werden im erfindungsgemäßen Verfahren die Zielmoleküle, bspw. Nukleinsäuren, mit geeigneten Fluorophoren ein oder mehrfach fluoreszenzmarkiert (bspw. FITC, Texas-Rot, Cyanine, wie Cy3, Cy5, usw.). In diesem Fall ist daher das zu erwartende Signal ein Fluoreszenz-Signal, das mit einem für Mikroarrays geeigneten Fluorimeter, einem entsprechend ausgelegten konfokalen Fluoreszenzmikroskop mit gekoppelter CCD-Kamera oder anderen für die Mikroarray-Fluoreszenzdetektion geeigneten Messvorrichtungen bestimmt wird. Andere Erwartungs- und Testsignale, die bei Mikroarraytechniken Verwendung finden, bspw. die Messung elektrischer Signale (insbesondere die Änderung von Stromflüssen), welche die Wechselwirkung zwischen Sonden- und Zielmolekülen z.B. im Trägermaterial und/oder über geeignete Marker, bspw. Redox-Marker, hervorruft, sind selbstverständlich ebenfalls für das erfindungsgemäße Verfahren nutzbar.

Des Weiteren wird gemäß Unterschritt (2) (bzw. obigem Schritt (c) des Herstellungsverfahrens der vorliegenden Erfindung) ein Mikroarray der ermittelten Testsondenmoleküle (vorzugsweise Oligonukleotide) auf einem entsprechenden Träger (vorzugsweise Glas, das bspw. durch Silanisierung funktionalisiert wurde) *in situ* in hoher Dichte synthetisiert. Diese *in situ* Synthese der Testsondenmoleküle kann auf verschiedene, einem Fachmann bekannte Art und Weise erfolgen, z.B. nach den Verfahren der bereits vorstehend im Zusammenhang mit der erfindungsgemäßen *in* situ Synthese-Vorrichtung aufgeführten Anbieter. Besonders bevorzugt erfolgt die *in situ* Synthese mit Hilfe der MAS-Technik, wobei in diesem Zusammenhang wieder auf die in WO 99/42813, WO 01/34847 und WO 02/04597 dargelegten Ausführungsformen Bezug genommen wird.

Die *in situ* Synthese der Testsondenmoleküle in hoher Dichte bedeutet gemäß bevorzugter Ausführungsformen der vorliegenden Erfindung, dass mehr als 1000, vorzugsweise mindestens 4000, verschiedene (Test-)Sondenmolekülspezies, direkt auf dem für den Mikroarray gewählten Substrat auf einer Fläche von bspw. etwa 1200 mm² bis etwa 3000 mm², synthetisiert werden. Bevorzugte Mikroarray-Maße sind dabei etwa 20 x 60 mm bis etwa 38 x 75 mm, insbesondere etwa 25 x 75 mm. Das MAS-Verfahren erlaubt z.B. eine *in situ* Synthese von Sondenmolekülen von mehr als 750.000 Sondenmolekülspezies (Features).

Gemäß Unterschritt (3) der bevorzugten Ausführungsform des Verfahrens der vorliegenden Erfindung wird ein Test des so hergestellten Testmikroarrays mit Zielmolekülen durchgeführt, wobei mindestens ein Testsignal aufgenommen wird. Für die aufzunehmenden Testsignale, insbesondere Fluoreszenzsignale, gilt das hinsichtlich des Erwartungssignals vorstehend dargelegte.

Im nächsten Unterschritt (4) der bevorzugten Ausführungsform des erfindungsgemäßen Verfahrens wird das aufgenommene Testsignal bzw. die aufgenommenen Testsignale mit dem festgelegten Erwartungssignal (bzw. den mehreren Erwartungssignalen) verglichen. Dieser Vergleich wird typischerweise mit Hilfe von bekannten Programmen mit Rechnern durchgeführt, in welche die erwarteten und gemessenen Signale eingelesen werden.

Aufgrund der Auswahl der Testsondenmoleküle, der Synthese der Sondenmoleküle *in situ* auf dem Träger und anderen mit Unsicherheiten behafteten Verfahrensvorgängen entspricht das zunächst im Unterschritt (3) erhaltene Testsignal nicht dem Erwartungssignal. Insbesondere ist bei Nukleinsäuren, bspw. Oligonukleotiden, als Sondenmolekülen zu beachten, dass bei der *in situ* Synthese keine 100%ige Ausbeute erreicht werden kann, weshalb einige Oligonukleotide im jeweiligen Spot nicht die erwartete bzw. ermittelte Länge aufweisen bzw. andere Sequenzen aufweisen, da andere Nukleotide statt dem richtigen Nukleotid während der *in situ* Synthese eingebaut wurden. Des Weiteren ist häufig die Menge der aufgetragenen Sondenmoleküle pro Spot nicht bzw. nicht optimal an die jeweilige Anwendung angepaßt.

Eine Vorgehensweise zur Durchführung des Tests gemäß Unterschritt (3) ist bspw., die aufgetragenen Sondenmoleküle, insbesondere DNA, zu markieren, insbesondere mit einem oder mehreren Fluoreszenzmarkern zu versehen, um dann eine Aufnahme eines Fluoreszenzbildes des Arrays bereitzustellen. Dazu muß nicht jedes aufgetragene Sondenmolekül, bspw. nicht jedes Oligonukleotid, markiert werden, sondern es reicht erfindungsgemäß aus, dass bspw. nur jedes 100ste, 500ste oder 1000ste Sondenmolekül markiert wird. Bspw. kann bei Oligonunkleotiden unter Verwendung eines 1000:1-Gemischs nicht-markierter und z.B. Fluorescein-markierter Nukleotide beim Start der Oligonukleotidsynthese gemäß Unterschritt (2) nur jedes 1000ste Oligonukleotid markiert werden. Die Markierung nur weniger Sondenmoleküle hat den Vorteil, dass keine erheblichen Kosten durch die Markierung auftreten. Des Weiteren werden so mögliche Fluoreszenzlöschungseffekte (z.B. Fluoreszenz-Resonanz-Energie-Transfer (FRET)) vermieden bzw. weniger wahrscheinlich. Idealerweise wird die Fluoreszenz-Markierung der im Mikroarray aufgetragenen Oligonukleotide derart eingestellt, dass die gemessene Fluoreszenz in etwa der Stärke der Fluoreszenz entspricht, die entsteht, wenn die aufgetragenen Sondenmoleküle mit markierten Zielmolekülen hybridisiert werden.

Gemäß einer weiteren bevorzugten Ausführungsform wird erfindungsgemäß der Test im Unterschritt (3) bei Oligonukleotiden als Testsondenmolekülen wie nachstehend dargelegt durchgeführt. Dieser Test dient im wesentlichen der Überprüfung, ob die ermittelten und im Array aufgetragenen Testsondenmoleküle tatsächlich mit Zielmolekülen zur Hybridisierung befähigt sind.

In einem ersten Schritt werden dazu die im Array aufgetragenen Oligonukleotide in Sätzen von etwa 1000 (z. B. 3 x 384 Vertiefungen pro Mikrotiterplatte) aufgeteilt. In einem zweiten Schritt wird eine 10 bis 15 Nukleotide umfassende Sequenz an das 3'-Ende der gesammelten Oligonukleotide ligiert. Mit dieser Verlängerung wird eine komplementäre Nukleotidsequenz (ebenfalls 10 bis 15 Nukleotide umfassend) hybridisiert. Danach wird die komplementäre Basensequenz (Zweitstrang) unter Verwendung von Taq-Polymerase verlängert. Danach wird ein Denaturierungsschritt zur Trennung von Matrizen- und Zweitstrang durchgeführt. Die Schritte der Hybridisierung, Verlängerung und Denaturierung werden wie bei einer PCR wiederholt, um einen markierten Zweitstrang im Überschuß gegenüber dem Matrizenstrang zu erzeugen. Schließlich wird der so erzeugte Zweitstrang mit einem Fluorophor (end-)markiert und mit dem Mikroarray hybridisiert.

Durch dieses Verfahren wird beim Messen der Fluoreszenzen ersichtlich, welche Oligonukleotide nur ineffizient oder gar nicht hybridisieren. Diese können dann ggf. verändert und erneut getestet werden. Bei einer anderen Ausführungsform dieser Vorgehensweise kann der Erststrang über Biotin an eine mit Streptavidin überzogene Mikrotiterplatte gebunden werden, um sowohl die Reinigung des Zweitstrangs als auch die Wiederverwendung der Matrize zu erleichtern. Man kann ebenfalls eine 10 bis 15 Nukleotide umfassende komplementäre Sequenz, die bereits am 5'-Ende mit einem Fluorophor versehen ist, synthetisieren, was das Erfordernis der Markierung nach der Zweitstrangsynthese umgeht. Des Weiteren ist es möglich, einen vierten Fluorophor für dieses Kontrollmaterial zu verwenden und zusammen mit den eigentlichen, bspw. mit Cy3- und Cy5-markierten Zielmolekülen bei jeder Verwendung des Mikroarrays zu hybridisieren. Dies gewährleistet, dass die Hybridisierung mit den Mikroarray-Sondenmolekülen für jede Produkteinheit gleich bleibt.

Entsprechend des Vergleichs zwischen Test- und Erwartungssignal wird erfindungsgemäß der Mikroarray durch Wiederholen der Unterschritte (2) bis (4) entsprechend angepaßt, bis das oder die Testsignal(e) im wesentlichen dem/den Erwartungssignal(en) entspricht/entsprechen. Dabei werden im Unterschritt (2) die Parameter des Mikroarray-Layouts jeweils entsprechend der Ergebnisse des Tests im Unterschritt (3) und des Vergleichs gemäß Unterschritt (4) der jeweils vorhergehenden Runde verändert, d.h. es werden die Testsondenmoleküle, z.B. im Fall von Nukleinsäuren, wie Oligonukleotiden, deren Sequenz, Länge, Markierung, Art (z.B. Verwendung von Nukleotidanaloga anstatt von natürlich vorkommenden Nukleotiden), deren Menge, Anzahl, Dichte, Bindung auf dem Substrat, Orientierung und/oder Anordnung auf dem Mikroarray verändert. Weitere veränderliche Parameter können auch die Reaktionsbedingungen, bei Nukleinsäuren als Sonden- und Zielmolekülen insbesondere die Hybridisierungsbedingungen, wie Temperatur, pH, Ionenstärke, damit zusammen hängend insbesondere Lösungs- bzw. Pufferzusammensetzung, z.B. Salzgehalt, Gehalt an Detergentien usw. betreffen. Die Schritte Herstellen des Testmikroarrays (ggf. mit veränderten Parametern) (Unterschritt (2)), Testen des Mikroarrays mit Zielmolekülen (Unterschritt (3)) und Vergleichen von Testsignalen und Erwartungssignalen (Unterschritt (4)) werden solange unter entsprechender Anpassung des Mikroarrays wiederholt, bis das oder die Testsignal(e) im wesentlichen dem/den Erwartungssignal(en) entspricht/entsprechen.

Bei der Validierung des (Test-)Mikroarrays ist es dabei weiter bevorzugt, die beim Testen und Auslesen des Testmikroarrays gewonnenen Daten bei jeder oder auch nach mehreren Anpassungsrunden dem Anwender, der die Informationen vorzugsweise unter Verwendung der erfindungsgemäßen Eingabe-Einrichtung über die Zielmoleküle in das System eingespeist hat, zu übertragen. Anhand dieser Daten kann dann wiederum der Anwender in den Validierungsprozess eingreifen und dabei auch möglicherweise in der Zwischenzeit gewonnene weitere Erkenntnisse (eigene experimentelle Daten, Literaturdaten usw.) über die jeweiligen Zielmoleküle und/oder die zum jeweiligen Zeitpunkt im Test befindlichen Sondenmoleküle und/oder die bei der Wechselwirkung einzuhaltenden Reaktionsbedingungen dem weiteren Validierungsprozess zur Verfügung stellen. Bevorzugt wird dabei wieder der elektronische Datentransfer über die vorstehend erläuterten Einrichtungen, Netzwerke usw. verwendet. Mit dieser bevorzugten Variante des erfindungsgemäßen Verfahrens wird ein schleifenartiger Prozess bereitgestellt, der durch die Verbreiterung der Informationsbasis über die im jeweiligen Stadium befindlichen Sondenmoleküle, Zielmoleküle und die im Test zu wählenden Bedingungen, in besonders effizienter Weise ein optimiertes Layout und damit einen fehlerfreien, maßgeschneiderten Mikroarray bereitstellt.

Im letzten Unterschritt (6) der bevorzugten Ausführungsform des Verfahrens der vorliegenden Erfindung wird das Layout des derartig validierten Mikroarrays bereitgestellt, bei dem das Testsignal bzw. die Testsignale im wesentlichen dem/den Erwartungssignal(en) entspricht/entsprechen. Dies kann wiederum in Form eines Daten-Files dem Mikroarray-Anwender, der vorzugsweise bei einem Sondenmolekül-Hersteller angeordneten Sondenmolekülsynthese-Einrichtung und/oder der vorzugsweise bei einem Mikroarray-Hersteller angeordneten Mikroarray-Bereitstellungseinrichtung mit Hilfe einer elektronischen Datenübertragungseinrichtung bereitgestellt werden, wobei diesbezüglich die vorstehenden Ausführungen bezüglich Schritt (a) des erfindungsgemäßen Verfahrens analog gelten. So werden z.B. die Informationen über das ermittelte Mikroarray-Layout in einem (oder mehreren Daten-File(s) abgelegt und vorzugsweise über einen Zentralrechner per E-mail an bei einem Sondenmolekül-Hersteller (insbesondere Oligonukleotid-Hersteller) und bei einem Mikroarray- oder Biochip-Hersteller dezentral angeordnete Computer übertragen.

Die *ex situ* Sondenmolekülsynthese erfolgt entsprechend des im vorhergehenden Schritt bereitgestellten Layouts, vorzugsweise nach im Stand der Technik beschriebenen Festphasensynthese-Verfahren, insbesondere (wie bereits vorstehend im Zusammenhang mit dem System der vorliegenden Erfindung ausgeführt) nach Art der Merrifield-Synthese (H.G. Gassen et al. (1982), *supra;* Gait (1987), *supra*) oder auf andere Art (Beyer und Walter (1984), *supra*). Bevorzugte Synthese-Verfahren bei Oligonukleotiden beruhen auf der Phosphoramidit-Methode und verwenden typischerweise (vorzugsweise Aminoakyl-modifizierte, insbesondere Aminopropyl-modifizierte) poröse Glasträger (CPG, engl. "Controlled Pore Glass") Derartige Verfahren weisen den Vorteil auf, dass ein gegebenes (validiertes) Sondenmolekül bzw. eine gegebene (validierte) Sondenmolekülspezies (Feature), z.B. ein Oligonukleotid gegebener Sequenz, vorzugsweise mit einer Länge von etwa 8 bis etwa 80 Nukleotiden, etc., in großer Menge bei niedrigen Kosten in relativ kurzer Zeit und guter Ausbeute bereitgestellt werden kann.

Die Bereitstellung des erfindungsgemäßen Endprodukts (validierter Mikroarray) erfolgt wiederum entsprechend des im Schritt (c) des Verfahres der vorliegenden Erfindung ermittelten Layouts durch Aufbringen der *ex situ* synthetisierten Sondenmoleküle auf ein entsprechendes Substrat. Erfindungsgemäß verwendbare Vorgehensweisen zum Aufbringen von *ex situ* synthetisierten Sondenmolekülen sind Stand der Technik und umfassen z.B. entsprechende Spotting-Methoden (die z.B. auch als "Druck-Methoden" oder Inkjet-Methoden" bezeichnet werden).

Durch die vorstehend dargelegten Schritte wird demnach ein validierter Mikroarray bereitgestellt, welcher der ausgehenden Problemstellung, insbesondere genomischen Problemstellungen bei Verwendung von DNA-Mikroarrays, so gut wie möglich angepasst und daher als besonders fehlerarm einzustufen ist.

Gemäß einer weiteren bevorzugten Ausführungsform der vorliegenden Erfindung werden mehrere Mikroarrays auf ein Substrat aufgebracht, so dass man von einer sogenannten "Array of Array"- oder "multiplen" bzw. "multiplexed" Array-Anordnung spricht. Bei einer multiplen Array-Anordnung können selbstverständlich Mikroarrays mit jeweils gleichem oder unterschiedlichem Design vorliegen.

Dazu wird vorzugsweise mit Hilfe geeigneter hydrophober Agenzien, insbesondere entsprechender hydrophober Polymere oder Polymerzusammensetzungen (bspw. Silikone, Teflon oder ander polyfluorierte Polymere- oder Polymerzusammensetzungen, z.B. Produkte von Cytonix wie PerFluoroCoat™), einem Substrat ein hydrophobes Muster (sog. "Patterning") aufgeprägt. Hierzu sind im Stand der Technik geeignete Verfahren bekannt, wobei erfindungsgemäß mit entsprechenden Polymerzusammensetzungen durchgeführte Siebdruckverfahren bevorzugt sind. Hierdurch können auf einem Substrat, bspw. einem Glasträger, vorzugsweise mit den Dimensionen 25 x 75 mm (entsprechend den Maßen üblicher Objektträger) z.B. 2, 8 (2 x 4) 12 (2 x 6), 16 (2 x 8) 48 (4 x 12) oder 192 (8 x 24) Reaktionskammern oder -bereiche (Vertiefungen oder "Wells") erzeugt werden, die jeweils von der hydrophoben Beschichtung umgeben und dadurch voneinander abgegrenzt sind. Selbstverständlich ist es ebenfalls möglich, entsprechende Reaktionsbereiche in ein geeignetes Substrat zu stanzen statt eine Beschichtung vorzunehmen. Jeder Reaktionsbereich ist dabei zur Aufnahme eines erfindungsgemäßen Mikroarrays ausgelegt. Selbstverständlich sind das Design, insbesondere die Anzahl, Form (rund, oval, eckig) und Anordnung, der Reaktionsbereiche auf dem Substrat, und die Dimensionen des Substrats vollkommen frei wählbar und können individuell den jeweiligen Bedürfnissen des Anwenders angepasst werden. Bspw. können auch größere oder kleinere Substratdimensionen als die vorstehend genannten gewählt werden. Als ein bevorzugtes Beispiel kann ein Substrat mit den Maßen üblicher Mikrotiterplatten (bspw. 85,48 x 127,76 mm) genannt werden. Derartige multiple Array-Anordnungen weisen den Vorteil auf, dass sie in besonders einfacher Weise in übliche automatisierte Systeme zur Flüssigkeitshandhabung und Hochdurchsatzverfahren integriert werden können.

Die Vorteile multipler Array-Anordnungen liegen insbesondere in einer Erhöhung der Reproduzierbarkeit (sämtliche Mikroarrays auf demselben Substrat werden unter idenitischen Bedingungen aufgebracht und mit den Zielmolekülen in Kontakt gebracht (z.B. hybridisiert); Extraktion, reverse Transkription, Amplifizierung und Markierung von Zielmolekülen kann ebenfalls für jeden Mikroarray gleichzeitig erfolgen; Möglichkeit der mehrmaligen Charakterisierung eines einzelnen Zielmoleküls auf einem einzigen Substrat (Verbesserung der Statistik)), der Verminderung von Kosten (der Kostenanstieg ist typischerweise proportional zu 1/Anzahl der Mikroarrays, so liegen üblicherweise die Kosten einer multiplen Array-Anordnung bei kleiner 5fach gegenüber einem einzelnen Mikroarray, jedoch kann die multiple Array-Anordnung zur Charakterisierung von bspw. 8 bis 200 unterschiedlichen Testansätzen verwendet werden) und der Erhöhung des Durchsatzes (reduzierte Probenhandhabung, da multiple Array-Anordnungen gleichzeitig mit Testlösungen in Kontakt gebracht (z.B. hybridisiert) werden; Möglichkeit der Automatisierung, insbesondere bei Verwendung von Substraten im Mikrotiterplattenformat).

Weiterhin können erfindungsgemäße Mikroarrays oder Array of Array-Anordnungen in vorteilhafter Weise mit adhesiven Superstrukturen (bspw. von Grace Bio-Labs, Inc., Bend, OR, USA, im Handel erhältlich) versehen werden, um so das Verdampfen von Lösungsmittel (meist Wasser bzw. Puffer; insbesondere beim Testen von Mikroarrays mit geringen Probenvolumina von Bedeutung) und eine Kontamination benachbarter Mikroarrays (bei multiplen Array-Anordnungen) zu minimieren.

Die erfindungsgemäßen multiplen Array-Anordnungen können sowohl bei der Bereitstellung des validierten Layouts für die erfindungsgemäßen validierten Mikroarrays (gemäß Schritt (c) des erfindungsgemäßen Verfahrens) als auch beim Aufbringen der validierten Sondenmoleküle (gemäß Schritt (e) des Verfahrens der vorliegenden Erfindung) auf ein (entsprechend mit einem, vorzugsweise hydrophoben, Patterning versehenen) Substrat zum Einsatz kommen. Bevorzugt ist die Bereitstellung erfindungsgemäß validierter Mikroarrays in multiplen Array-Anordnungen, da sich die entsprechenden Verfahren zum Aufbringen der validierten Sondenmoleküle (z.B. Spotting, Kontaktdruck usw.) hierfür am besten eignen. Somit umfassen die genannten Schritte (Schritt (c) und/oder Schritt (e), vorzugsweise Schritt (e)) des erfindungsgemäßen Vefahrens gemäß dieser bevorzugten Ausführungsform das Herstellen von mehr als einem Testmikroarray (siehe Schritt (c) des erfindungsgemäßen Verfahrens) bzw. das Aufbringen von validierten Sondenmolekülen in mehreren Mikroarrays (siehe Schritt (e) des erfindungsgemäßen Verfahrens). Dabei können die noch nicht validierten Testmikroarrays (Schritt (c)) bzw. die Mikroarrays mit den validierten Layouts (Schritt (e)) jeweils gleich oder unterschiedlich sein. Die Aufteilung des Substrats erfolgt durch im Stand der Technik bekannte Patterning-Vorrichtungen, vorzugsweise durch zum Inkjet-Printing ausgelegte Siebdruckgeräte (bspw. erhältlich von Systematic Automation, Inc., Farmington, CT, USA). Erfindungsgemäß ist es, wie bereits vorstehend erläutert, auch beim Design erfindungsgemäßer Mikroarrays in multiplen Array-Anordnungen bevorzugt, vom Anwender im Rahmen des Schritts (c) des erfindungsgemäßen Verfahrens ermittelte oder vorgegebene Informationen hinsichtlich der Gestaltung der multiplen Anordnung in die Entwicklung des herzustellenden Endprodukts (hier: mehrere Mikroarrays in einer multiplen Arraystellenden Endprodukts (hier: mehrere Mikroarrays in einer multiplen Array-Anordnung) einfließen zu lassen.

Ein weiterer Gegenstand der vorliegenden Erfindung betrifft die durch das erfindungsgemäße Verfahren erhältlichen validierten Mikroarrays (d.h. Mikroarrays mit validiertem Layout). Diese können gemäß einer bevorzugten Ausführungsform in einer multiplen Array-Anordnung, wie vorstehend beschrieben, vorliegen.

Die Figuren zeigen:

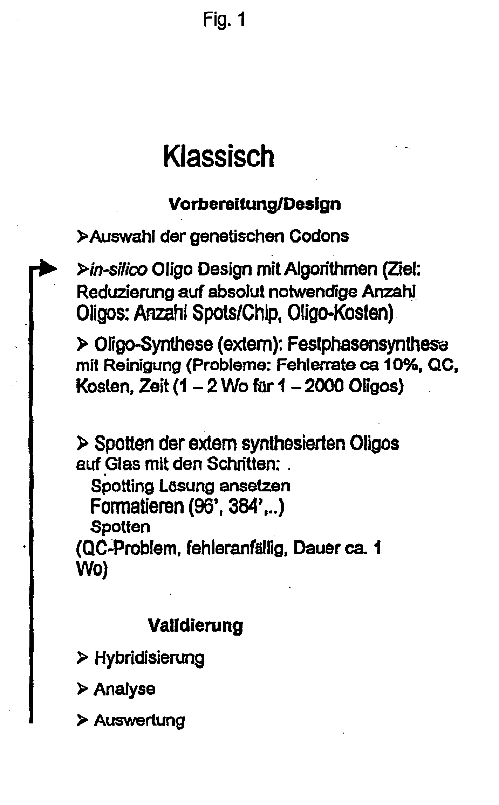

- Fig. 1: zeigt das bisher übliche Vorgehen zur Herstellung validierter Mikroarrays am Beispiel von DNA-Mikroarrays. Der erste Abschnitt üblicher Verfahren betrifft die Vorbereitung und das Design des DNA-Mikroarrays. Dazu werden zunächst die Codons ausgewählt. Es folgt ein *in silico* Oligonukleotid-Design durch bekannte Algorithmen mit dem Ziel, die Anzahl der benötigten Oligonukleotide auf die minimal erforderliche Anzahl zu reduzieren. Weitere Parameter betreffen die Optimierung der Anzahl der Spots pro Chip. Diese Optimierungen dienen im wesentlichen der Reduzierung von Kosten, die bei der nachfolgenden Oligonukleotid-Synthese anfallen. Die Oligonukleotid-Synthese erfolgt meist extern vom Anwender mittels Festphasen-Synthese, die häufig einen Reinigungsschritt umfaßt. Dabei ist von einer Fehlerrate der Synthese von etwa 10% auszugehen, was einen entsprechenden Qualitätscheck bzw. eine entsprechende Qualitätskontrolle erforderlich macht. Dadurch entstehen weitere hohe Kosten und es ergeben sich allein für diese Oligonukleotid-Synthese Zeitspannen für ein bis zwei Wochen (für bspw. 1000 bis 2000 Oligonukleotide). Die derart extern synthetisierten Oligonukleotide müssen auf einen entsprechenden Träger, insbesondere Glas, aufgetragen (gespottet) werden. Das Spotten umfaßt insbesondere die Schritte des Herstellens des Spotting-Lösung, das Formatieren entsprechend dem Mikroarray in Mikrotiterplatten mit 96, 384 usw. Vertiefungen und das Spotten der Lösungen selbst. Auch hier ergeben sich entsprechende Qualitätskontrollprobleme, da die einzelnen Schritte aufgrund der Vielzahl von anzusetzenden Lösungen etc. relativ fehleranfällig sind. Darüber hinaus muß für einen gängigen Mikroarray etwa eine Woche für diesen Schritt des Spottings angesetzt werden. Danach muß der so hergestellte Mikroarray durch die Schritte Hybridisierung, Analyse und Auswertung validiert werden. Dabei ist aufgrund der bereits mit Fehlern behafteten Auswahl der Oligonukleotide durch die Algorithmen das gesamte Verfahren ab der *in silico* Oligonukleotidauswahl zu wiederholen, bis das Design (Layout) des Mikroarrays den Anforderungen entspricht, was Wochen bis Monate in Anspruch nimmt.

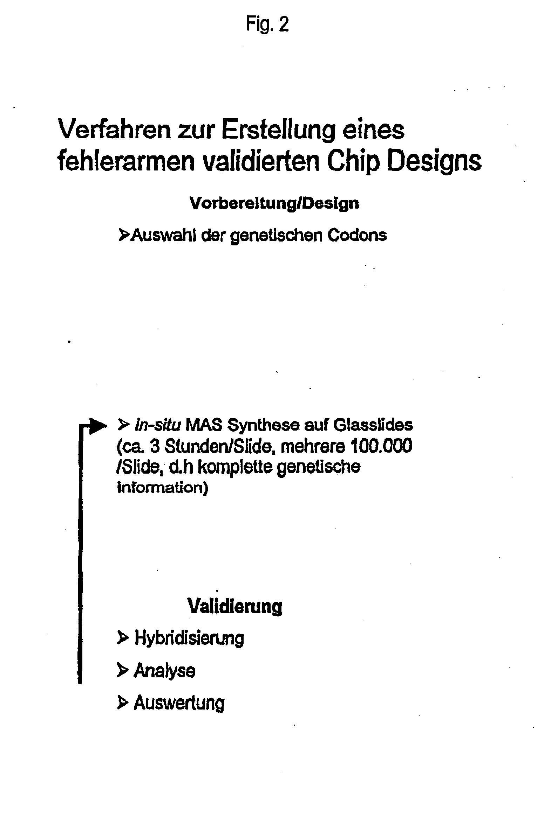

- Fig. 2: zeigt im Gegensatz dazu das Vorgehen gemäß dem Verfahren der vorliegenden Erfindung, bei dem zunächst die Codons ausgewählt werden (anhand der vom Anwender bereitgestellten Informationen über die Zielnukleinsäuren), wonach die *in situ* MAS-Synthese der ausgewählten Sondenoligonukleotide auf Glasträgern (Glasslides) erfolgt. Dieses Syntheseverfahren ist extrem schnell (etwa drei Stunden/Slide) und kann auch bei extrem umfangreichen Mikroarrays durchgeführt werden (mehrere 100 000 Oligos/Slide). Dies bedeutet, dass ggf. auch die ermittelten und aufgetragenen Sondenoligonukleotide die vollständige genetische Information eines Organismus repräsentieren können. Danach erfolgt die Validierung des zunächst bereitgestellten Mikroarrays durch die Unterschritte Hybridisierung, Analyse und Auswertung, wobei ggf. wieder eine geänderte Synthese unter Anpassung bspw. der Länge und Sequenz der Oligonukleotide durchgeführt wird. Die Auswahl der Sondenoligonukleotide erfolgt bezüglich Spezifität und der Darstellung des erforderlichen Mikroarray-Layouts (Chip-Designs) entsprechend der vom Anwender, insbesondere vom Hersteller des erwünschten Mikroarrays (Chips), bereitgestellten Informationen, die vorzugsweise per Internet bereitgestellt werden. Danach erfolgt die Synthese der identifizierten Oligonukleotide gemäß dem validierten Layout durch übliche Festphasen-Syntheseverfahren sowie das Aufbringen der validierten Sondenmoleküle gemäß dem validierten Layout durch Spotting- oder Druckverfahren bei einem Mikroarray-Hersteller. Die wesentlichen Vorteile dieser Vorgehensweise liegen zum einen in der enormen Zeiteinsparung von einem Zeitaufwand von (je nach Umfang der zur Verfügung stehenden Informationen) etwa 5 bis 10 Wochen beim konventionellen Vorgehen zur Erstellung des Layouts auf bis zu 1 Tag bis 1 Woche (Bereitstellung der Informationen über die zu untersuchenden Zielmoleküle bis zum validierten MikroarrayLayout), wobei darüber hinaus beim erfindungsgemäßen Verfahren erhebliche Kosteneinsparungen durch den Einsatz üblicher, für die Massenproduktion besonders geeigneter Verfahren bei der Herstellung des validierten Mikroarrays resultieren.

Die Anwendungsmöglichkeiten des erfindungsgemäßen Systems und des Verfahrens sind insbesondere bei der Herstellung spezifischer Mikroarrays, insbesondere DNA-Mikroarrays, zur Wirkstoffauswahl vorgesehen. Des Weiteren können die mittels dem erfindungsgemäßen Verfahren, insbesondere unter Verwendung des erfindungsgemäßen Systems, bereitgestellten Mikroarrays bei der patientenspezifischen Wirkstoffauswahl eingesetzt werden. Hier kommt es erfahrungsgemäß auf die Geschwindigkeit an, in der ein Patient das für sein Krankheitsbild optimierte Arzneimittel erhält. Des Weiteren können Mikroarrays, welche auf Grundlage des erfindungsgemäß hergestellten Layouts, bereitgestellt werden, als Basis für Toxizitätstests dienen, welche ebenfalls eine kurze Reaktionszeit erfordern. Des Weiteren können erfindungsgemäß bereitgestellte Mikroarrays auch zur Evaluierung und Optimierung von Herstellungsanlagen für biotechnologisch erzeugte Produkte herangezogen werden. Derartige Anlagen werden meist über eine On-line-Prozesskontrolle überwacht, wobei die biologische Aktivität der zur Herstellung der gewünschten Produkte herangezogenen Bakterien optimiert wird.

## Patentansprüche

1. System zur entkoppelten Herstellung validierter Mikroarrays mit mindestens
- einer oder mehreren Einrichtung(en), die zur Eingabe von Informationen über durch einen Mikroarray zu analysierende Zielmoleküle ausgelegt ist/sind,
- einer Einrichtung, die zum Ermitteln von potentiell mit den Zielmolekülen wechselwirkenden Sondenmolekülen (Testsondenmoleküle) anhand von Informationen über zu analysierende Zielmoleküle ausgelegt ist,
- einer Layout-Bereitstellungseinrichtung, umfassend
- eine Vorrichtung zur *in situ* Synthese von Testsondenmolekülen in hoher Dichte,
- eine Mikroarray-Testvorrichtung, die zum Inkontaktbringen von Zielmolekülen mit Testsondenmolekülen ausgelegt ist, und
- eine Mikroarray-Auslesevorrichtung, die zur Aufnahme von beim Inkontaktbringen von Zielmolekülen und Testsondenmolekülen veränderlichen Signalen ausgelegt ist,
- einer Sondenmolekülsynthese-Einrichtung, die zur *ex situ* Synthese von Sondenmolekülen ausgelegt ist, und
- einer Mikroarray-Bereitstellungseinrichtung, die zum Aufbringen von Sondenmolekülen auf Substrate ausgelegt ist.

2. System nach Anspruch 1, wobei die Einrichtung(en) zur Eingabe von Informationen über durch einen Mikroarray zu analysierende Zielmoleküle (jeweils) einen Rechner umfasst, der die Informationen in einem oder mehreren Daten-File(s) ablegt.

3. System nach Anspruch 1 oder 2, wobei die Einrichtung zur Ermittlung von Testsondenmolekülen einen Rechner umfasst, der die Testsondenmoleküle programmgesteuert anhand von Informationen über Zielmoleküle auswählt.

4. System nach einem der Ansprüche 1 bis 3, wobei die Layout-Bereitstellungseinrichtung einen Rechner umfasst, der das Layout in Form eines Daten-Files ablegt.

5. System nach einem der Ansprüche 1 bis 4, wobei die Sondenmolekülsynthese-Einrichtung eine Festphasensynthese-Vorrichtung umfasst.

6. System nach einem der Ansprüche 1 bis 5, wobei die Mikroarray-Bereitstellungseinrichtung eine Spotting-Vorrichtung umfasst.

7. System nach einem der Ansprüche 1 bis 6, wobei die Mikroarray-Bereitstellungseinrichtung und/oder die Layout-Bereitstellungseinrichtung eine Patterning-Vorrichtung umfasst, die zur Aufteilung von Substraten in mehrere Reaktionsbereiche ausgelegt ist.

8. System nach einem der Ansprüche 1 bis 7, wobei die Einrichtungen über Datenübertragungsvorrichtungen in einem Netzwerk verbunden sind.

9. System nach Anspruch 8, wobei das Netzwerk das Internet ist.

10. System nach Anspruch 8 oder 9, wobei die Einrichtungen in einer Plattform für Interaktionen in einem Forschungskonsortium zusammengeschlossen sind.

11. Verfahren zur entkoppelten Herstellung eines validierten Mikroarrays mit dem System nach einem der vorhergehenden Ansprüche, umfassend die Schritte:
(a) Bereitstellen von Informationen über durch den Mikroarray zu analysierende Zielmoleküle durch die Eingabe-Einrichtung,
(b) Ermitteln von Testsondenmolekülen anhand der Informationen durch die Einrichtung zur Ermittlung von Testsondenmolekülen,
(c) Bereitstellen eines validierten Layouts für den Mikroarray durch die Layout-Einrichtung, umfassend das
- Herstellen mindestens eines Testmikroarrays durch *in situ* Synthese der ermittelten Testsondenmolekülen in hoher Dichte durch die *in situ* Synthese-Vorrichtung,
- Testen des Testmikroarrays mit Zielmolekülen durch die Mikroarray-Testvorrichtung und
- Aufnahme mindestens eines beim Testen des Testmikroarrays mit den Zielmolekülen veränderlichen Signals durch die Mikroarray-Auslesevorrichtung,
(d) Synthetisieren der dem validierten Layout entsprechenden Sondenmoleküle (validierte Sondenmoleküle) *ex situ* durch die Sondenmolekülsynthese-Einrichtung, und
(e) Aufbringen der validierten Sondenmoleküle gemäß dem validierten Layout auf ein Substrat durch die Mikroarray-Bereitstellungseinrichtung.

12. Verfahren nach Anspruch 11, wobei die Bereitstellung der Informationen im Schritt (a) und/oder das Bereitstellen des validierten Layouts im Schritt (c) (jeweils) in Form von Daten-Files mit Hilfe einer elektronischen Datenübertragungseinrichtung erfolgt.

13. Verfahren nach einem der Anspruch 11 oder 12, wobei die Ermittlung der Testsondenmoleküle im Schritt (b) mit Hilfe von Computerprogrammen erfolgt.

14. Verfahren nach einem der Ansprüche 11 bis 13, wobei das Bereitstellen des validierten Layouts im Schritt (c) die folgenden Unterschritte umfasst:
(1) Festlegen von mindestens einem bei einer Wechselwirkung der Zielmoleküle mit Sondenmolekülen von einem Mikroarray zu erwartenden Signal (Erwartungssignal),
(2) Herstellen eines Testmikroarrays durch *in situ* Synthese der ermittelten Testsondenmoleküle in hoher Dichte,
(3) Testen des Testmikroarrays mit Zielmolekülen, wobei mindestens ein Testsignal aufgenommen wird,
(4) Vergleichen des mindestens einen Testsignals mit dem mindestens einen Erwartungssignal,
(5) Anpassen des Testmikroarrays durch Wiederholung der Schritte (2) bis (4) bis das Testsignal im wesentlichen dem Erwartungssignal entspricht, wobei im Schritt (2) die Testsondenmoleküle, deren Menge, Bindung, Anzahl, Dichte, Orientierung und/oder Anordnung auf dem Mikroarray verändert werden bzw. wird, und
(6) Bereitstellen des Mikroarray-Layouts, bei dem das mindestens eine Testsignal im wesentlichen dem mindestens einen Erwartungssignal entspricht.

15. Verfahren nach Anspruch 14, wobei das Erwartungssignal und das Testsignal Fluoreszenzsignale sind.

16. Verfahren nach einem der Ansprüche 11 bis 15, wobei im Schritt (b) die ermittelten Testsondenmoleküle eine Gesamtpopulation von mit den Zielmolekülen potentiell wechselwirkenden Molekülen repräsentieren.

17. Verfahren nach einem der Ansprüche 11 bis 16, wobei die Test- bzw. validierten Sonden- und/oder Zielmoleküle Nukleinsäuren, Peptide, Peptidnukleinsäuren, Polypeptide, Oligosaccharide, Polysaccharide und/oder niedermolekulare organische Substanzen sind.

18. Verfahren nach Anspruch 17, wobei die Test- bzw. validierten Sondenmoleküle Oligonukleotide sind.

19. Verfahren nach Anspruch 18, wobei die Oligonukleotide eine Länge von etwa 8 bis etwa 80 Nukleotide aufweisen.

20. Verfahren nach Anspruch 18 oder 19, wobei die Oligonukleotide in Schritt (b) das Gesamtgenom eines Organismus, das genetische Material eines oder mehrerer Chromosomen eines Organismus, eine cDNA-Bibliothek, Splice-Varianten, Promotorregionen, SNPs, Mutationen oder hochrepetitive DNA-Bereiche repräsentieren.

21. Verfahren nach einem der Ansprüche 11 bis 20, wobei die Zielmoleküle ein- oder mehrfach fluoreszenzmarkiert sind.

22. Verfahren nach einem der Ansprüche 11 bis 21, wobei die *in situ* Synthese im Schritt (c) bzw. Unterschritt (2) durch ein elektrochemisches Verfahren und durch *in situ* Spotting von Sondenmolekülbausteinen erfolgt.

23. Verfahren nach einem der Ansprüche 11 bis 22, wobei im Schritt (c) bzw. Unterschritt (2) mehr als etwa 1000 Testsondenmolekülspezies pro etwa 25 mm x 75 mm synthetisiert werden.

24. Verfahren nach Anspruch 23, wobei mindestens etwa 4000 Testsondenmolekülspezies pro etwa 25 mm x 75 mm synthetisiert werden.

25. Verfahren nach einem der Ansprüche 11 bis 24, wobei im Schritt (c) mehrere Testmikroarrays auf einem Substrat hergestellt und/oder im Schritt (e) validierte Sondenmoleküle in mehreren Mikroarrays auf einem Substrat aufgebracht werden.

26. Validierter Mikroarray, erhältlich durch das Verfahren nach einem der Ansprüche 11 bis 25.
